# EUROPEAN PATENT APPLICATION

(11) **EP 4 756 284 A1**
(43) Date of publication of application: **10.06.2026**
(21) Application number: 24850957.2
(22) Date of filing: 05.08.2024
(51) Int. Cl.: F21V 3/00, F21V 3/02, A61L 2/10

(54) **LAMPSHADE, LAMP ASSEMBLY, VEHICLE-MOUNTED REFRIGERATOR AND VEHICLE**

(30) Priority: 09.08.2023 CN 202322143098 U
(71) Applicant: BYD Company Limited, Shenzhen, Guangdong 518118 (CN)
(72) Inventor: WANG, Shaowei, Shenzhen, Guangdong 518118 (CN); ZHU, Peng, Shenzhen, Guangdong 518118 (CN); GONG, Wenqiang, Shenzhen, Guangdong 518118 (CN)
(74) Representative: Taor, Simon Edward William
(86) International application number: PCT/CN2024/109739
(87) International publication number: WO 2025/031312

(57) **Abstract**

A lampshade, a lamp assembly, a vehicle-mounted refrigerator, and a vehicle are provided. The lampshade includes a lampshade body. The lampshade body has a first side and a second side that are opposite to each other. The lampshade includes a recessed structure that is recessed toward the first side. The recessed structure includes a transparent portion. The transparent portion may transmit light from a sterilization lamp disposed on the first side outward from the second side.

## Description

This application claims priority to Chinese Patent Application No. 202322143098.3, filed with the China National Intellectual Property Administration on August 9, 2023 and entitled "LAMPSHADE, LAMP ASSEMBLY, VEHICLE-MOUNTED REFRIGERATOR, AND VEHICLE", which is incorporated herein by reference in its entirety.

### TECHNICAL FIELD

This application relates to the technical field of lampshades, and in particular, to a lampshade, a lamp assembly, a vehicle-mounted refrigerator, and a vehicle.

### BACKGROUND

A vehicle-mounted refrigerator or a vehicle is usually equipped with a sterilization lamp assembly. The sterilization lamp assembly includes a sterilization lamp for sterilization and a lampshade. To implement a sterilization effect of the sterilization lamp, the lampshade is usually provided with a transparent window to allow ultraviolet light to pass through.

In a related technology, a user can directly see component constructions on the other side of a lampshade through the transparent window, which affects an aesthetic appearance of the vehicle-mounted refrigerator or the vehicle.

### SUMMARY

An objective of this application is to provide a lampshade, a lamp assembly, a vehicle-mounted refrigerator, and a vehicle, to solve the problem that an internal structure of an existing lampshade can be easily seen through a transparent portion of the existing lampshade, affecting an aesthetic appearance of the lampshade assembly.

To implement the objective of this application, in a first aspect, this application provides a lampshade. The lampshade includes a lampshade body. The lampshade body has a first side and a second side that are opposite to each other. The lampshade includes a recessed structure that is recessed toward the first side. The recessed structure includes a transparent portion. The transparent portion is capable of transmitting light from a sterilization lamp disposed on the first side outward from the second side.

In a possible implementation, the transparent portion is made of a colorless and transparent material.

In a possible implementation, the lampshade body is made of a light-transmitting but opaque material. The transparent portion is made of quartz glass or a cyclic block copolymer.

In a possible implementation, both the lampshade body and the transparent portion are made of colorless and transparent materials. The lampshade body is coated with a light-transmitting coating.

In a possible implementation, the lampshade body is made of a cyclic block copolymer. The light-transmitting coating is an anti-ultraviolet coating.

In a possible implementation, the lampshade body and the recessed structure are integrally formed.

In a possible implementation, the recessed structure further includes a support portion. The support portion is separately connected to the lampshade body and the transparent portion to support the transparent portion.

In a possible implementation, one end of the transparent portion is connected to the lampshade body, and the other end is connected to the support portion.

In a possible implementation, the lampshade body is constructed as a first plate body, the support portion is constructed as a second plate body, a first included angle B is formed between the first plate body and the second plate body, and 90°≤B≤160°.

In a possible implementation, the lampshade body is constructed as the first plate body, the transparent portion is constructed as a third plate body, a second included angle A is formed between the first plate body and the third plate body, and 60°≤A≤150°.

In a possible implementation, the lampshade further includes a plurality of snap-fit joints. The snap-fit joints are disposed on the first side of the lampshade body and fastened to the lampshade body.

In a second aspect, this application further provides a lamp assembly, including:
a base;
a sterilization lamp, connected to the base; and
a lampshade, where the lampshade is fastened to the base, a recessed structure is disposed on a side, close to the base, of the lampshade, the lampshade and the base jointly enclose a cavity, the sterilization lamp is disposed in the cavity, and light from the sterilization lamp is transmitted outward from a transparent portion of the lampshade; and
the lampshade includes a lampshade body, the lampshade body has a first side and a second side that are opposite to each other, the lampshade includes the recessed structure that is recessed toward the first side, the recessed structure includes the transparent portion, and the transparent portion is capable of transmitting light from the sterilization lamp disposed on the first side outward from the second side.

In a possible implementation, the lamp assembly further includes a circuit board. The circuit board is mounted in the cavity. The sterilization lamp is disposed on the circuit board.

In a possible implementation, the lampshade body is constructed as a first plate body. A third included angle C is formed between the circuit board and the lampshade body, and 10°≤C≤60°.

In a possible implementation, the recessed structure further includes a support portion. The support portion is connected to the lampshade body to support the transparent portion. The support portion is constructed as a second plate body. A fourth included angle D is formed between the circuit board and the support portion, and 60°≤D≤120°.

In a possible implementation, the lamp assembly further includes a light baffle. The light baffle is fastened to the circuit board. The light baffle is disposed between the sterilization lamp and the lampshade body.

In a possible implementation, one end of the light baffle is in contact with the transparent portion.

In a possible implementation, the light baffle includes a hook portion and a blocking portion connected to the hook portion. The hook portion is hooked to the circuit board.

In a third aspect, this application further provides a vehicle-mounted refrigerator. The vehicle-mounted refrigerator includes:
a refrigerator housing, where a refrigerating cavity is formed in the refrigerator housing, and the refrigerator housing is provided with a mounting hole; and
a lamp assembly, where the lamp assembly is mounted in the mounting hole,
the lamp assembly includes a lampshade, the lampshade includes a lampshade body, the lampshade body has a first side and a second side that are opposite to each other, the lampshade includes a recessed structure that is recessed toward the first side, the recessed structure includes a transparent portion, and the transparent portion is capable of transmitting light from a sterilization lamp disposed on the first side outward from the second side.

In a fourth aspect, this application further provides a vehicle. The vehicle includes a lampshade, a lamp assembly, or a vehicle-mounted refrigerator, where the lamp assembly includes a lampshade, or the vehicle-mounted refrigerator includes a lampshade; and
the lampshade includes a lampshade body, the lampshade body has a first side and a second side that are opposite to each other, the lampshade includes a recessed structure that is recessed toward the first side, the recessed structure includes a transparent portion, and the transparent portion is capable of transmitting light from a sterilization lamp disposed on the first side outward from the second side.

In the technical solutions of this application, the lampshade body of the lampshade has the first side facing the sterilization lamp and the second side facing outward. The lampshade body is provided with the recessed structure that is recessed toward the first side, so that a visual blind region may be formed on the second side of the lampshade body, and the transparent portion is disposed in the blind region. The light from the sterilization lamp can be transmitted out of the lampshade through the transparent portion. The user cannot clearly see, through the transparent portion from a normal perspective, component structures disposed on the first side of the lampshade.

### BRIEF DESCRIPTION OF DRAWINGS

To more clearly illustrate describe technical solutions in implementations of this application or a conventional technology, the drawings used in the description of the implementations or the conventional technology are briefly introduced below. Obviously, the drawings described below are only some implementations of this application. For persons of ordinary skill in the art, other drawings can be obtained from these drawings without creative efforts.
FIG. 1 is a diagram of a structure of a lampshade provided in a related technology.
FIG. 2 is a diagram of a structure of an embodiment of a lampshade provided in this application.
FIG. 3 is a cross-sectional view of a first embodiment of a recessed structure in FIG. 2.
FIG. 4 is a cross-sectional view of a second embodiment of a recessed structure in FIG. 2.
FIG. 5 is a cross-sectional view of a third embodiment of a recessed structure in FIG. 2.
FIG. 6 is a cross-sectional view of an embodiment of a lamp assembly provided in this application.
FIG. 7 is a diagram of a three-dimensional structure of a lamp assembly provided in this application.
FIG. 8 is a diagram of a three-dimensional structure of the lamp assembly in FIG. 7 after a lampshade is removed.
FIG. 9 is a diagram of a three-dimensional structure of a light baffle.
FIG. 10 is a cross-sectional view of an embodiment of a vehicle-mounted refrigerator provided in this application.
FIG. 11 is a diagram of a three-dimensional structure of the vehicle-mounted refrigerator in FIG. 10 after a lampshade is removed.

Reference numerals for a related technology:
10': lampshade;
1': lampshade body;
2': transparent window.

Reference numerals for this application:
10000: vehicle-mounted refrigerator, 11000: refrigerator housing, 12000: refrigerating cavity, 13000: mounting hole;
1000: lamp assembly;
100: lampshade, 101: lampshade body, 1011: first side, 1012: second side, 102: recessed structure, 1021: transparent portion, 1022: support portion, 10221: first support plate, 10222: second support plate, 103: snap-fit joint, 200: base, 201: slot;
300: cavity;
400: sterilization lamp;
500: illumination lamp;
600: circuit board;
700: light baffle, 701: blocking portion, 7011: first blocking portion, 7022: second blocking portion, 702: hook portion.

### DESCRIPTION OF EMBODIMENTS

The following clearly and completely describes the technical solutions in implementations of this application with reference to the accompanying drawings in implementations of this application. It is clear that the described implementations are merely some but not all of implementations of this application. All other implementations obtained by persons of ordinary skill in the art based on implementations of this application without creative efforts shall fall within the protection scope of this application.

It should be noted that when an assembly is referred to as "fastened" to another assembly, it may be directly on the other assembly or there may be an assembly therebetween. When an assembly is referred to as "connected" to another assembly, it may be directly connected to the other assembly or there may be an assembly therebetween.

Unless otherwise defined, all technical and scientific terms used in this application have the same meanings as commonly understood by persons skilled in the art to which this application pertains. Terms used in the specification of this application are merely intended to describe specific embodiments, but are not intended to limit this application. The term "and/or" as used in this application includes any and all combinations of one or more related listed items.

The following further describes some implementations of this application in detail with reference to the accompanying drawings. In the case of no conflict, the following embodiments and features in the embodiments may be combined with each other.

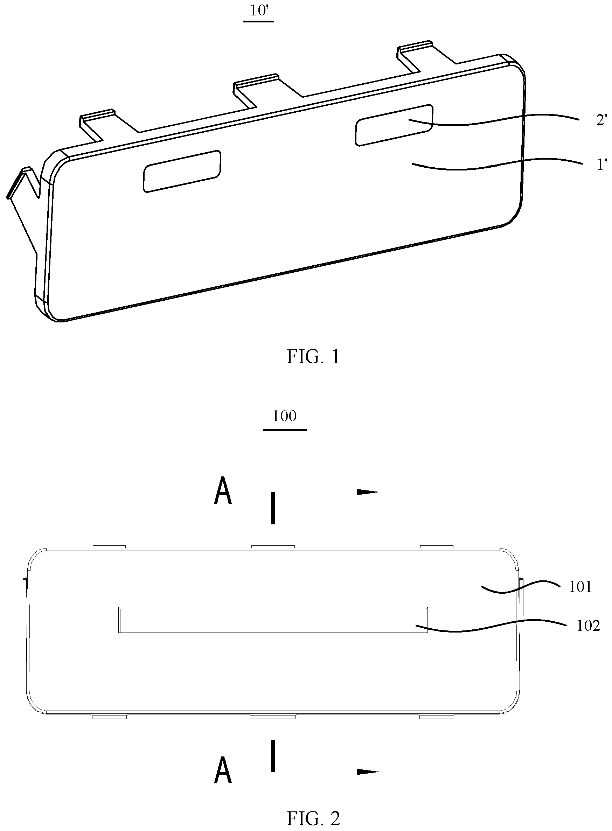

In a related technology, refer to FIG. 1. A transparent window 2' of a lampshade 10' and a lampshade body 1' are disposed in parallel. When a user looks at the lampshade 10', the user can directly see component constructions on the other side of the lampshade 10' through the transparent window 2', which affects an aesthetic appearance of a vehicle-mounted refrigerator.

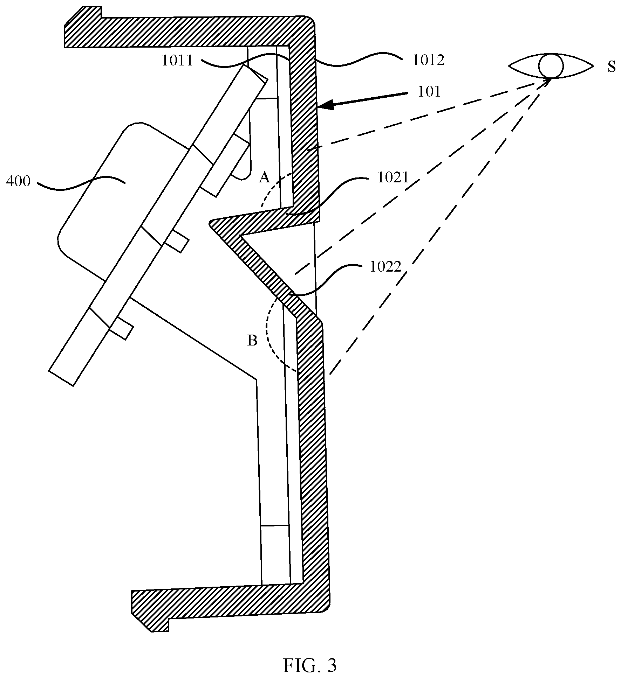

Refer to FIG. 2 and FIG. 3. To solve the foregoing problem, this application provides a lampshade 100. The lampshade 100 includes a lampshade body 101. The lampshade body 101 has a first side 1011 and a second side 1012 that are opposite to each other. The lampshade 100 includes a recessed structure 102 that is recessed toward the first side 1011. The recessed structure 102 includes a transparent portion 1021. The transparent portion 1021 is capable of transmitting light from a sterilization lamp 400 disposed on the first side 1011 outward from the second side 1012.

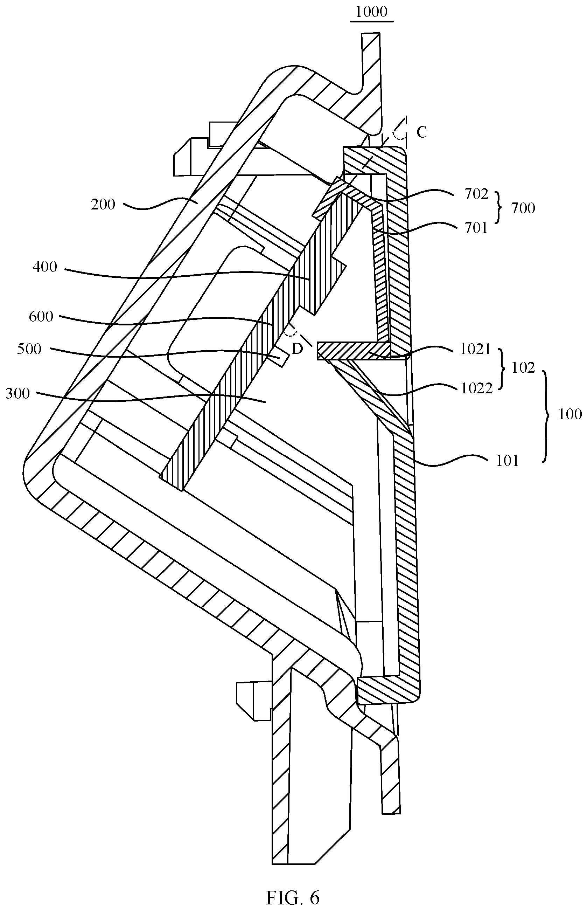

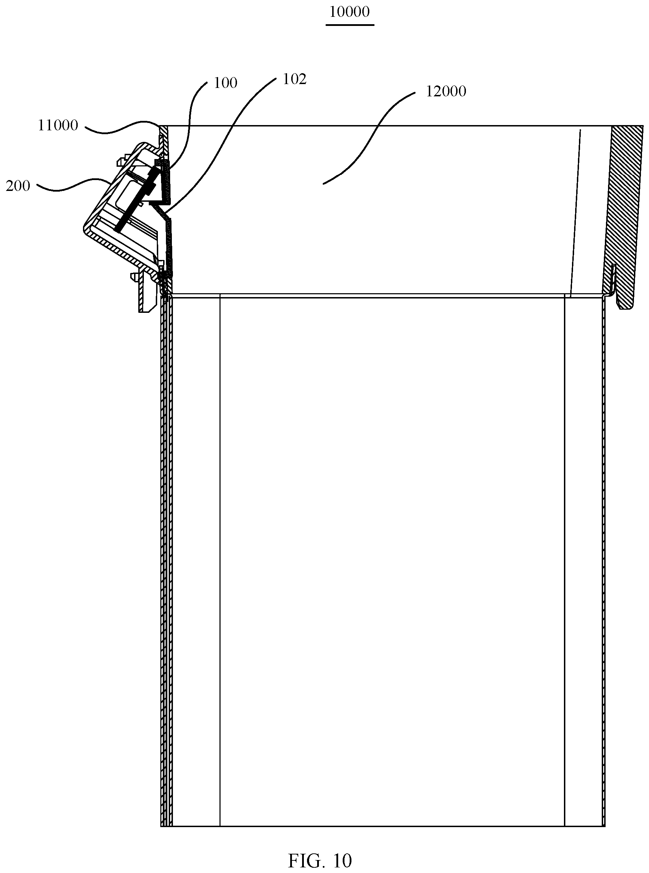

In the technical solutions of this application, the lampshade body 101 of the lampshade 100 has the first side 1011 facing the sterilization lamp 400 and the second side 1012 facing outward. The lampshade 100 is provided with the recessed structure 102 that is recessed toward the first side 1011, so that a visual blind region may be formed on the second side 1012 of the lampshade body 101, and the transparent portion 1021 is disposed in the blind region. The light from the sterilization lamp 400 can be transmitted out of the lampshade 100 through the transparent portion 1021. The user cannot clearly see, through the transparent portion 1021 from a normal perspective, component structures disposed on the first side 1011 of the lampshade body 101. This enhances an aesthetic appearance of the vehicle, the vehicle-mounted refrigerator 10000 (see FIG. 10), or the lamp assembly 1000 (see FIG. 6), which includes the lampshade 100, and improves user experience.

The lampshade 100 includes the lampshade body 101. The lampshade body 101 is configured to block the line of sight of the user, to prevent the user from seeing the component structures on the other side of the lampshade body 101 through the lampshade body 101, thereby enhancing an overall aesthetic appearance of an object provided with the lampshade 100.

The lampshade body 101 is provided with the recessed structure 102 for generating the visual blind region, and the transparent portion 1021 is disposed in the recessed structure 102. The recessed structure 102 further includes a support portion 1022. One end of the support portion 1022 is connected to the lampshade body 101, and the other end is connected to the transparent portion 1021. The support portion 1022 is configured to support the transparent portion 1021 and improve connection strength between the transparent portion 1021 and the lampshade body 101.

The support portion 1022 may be connected to any position of the transparent portion 1021, to provide support for the transparent portion 1021. In a possible implementation of this application, one end of the transparent portion 1021 is connected to the lampshade body 101, and the other end is connected to the support portion 1022. The support portion 1022 is connected to the farthest end of the transparent portion 1021 (an end, away from the lampshade body 101, of the transparent portion 1021), so as to minimize blocking of the transparent portion 1021 by the support portion 1022, and increase a light-transmitting area of the transparent portion 1021.

The lampshade body 101, the transparent portion 1021, and the support portion 1022 may be made of various materials. For example, in a possible implementation of this application, the lampshade body 101 is made of a light-transmitting but opaque material, so as to block the line of sight of the user while allowing the light to pass through. The light-transmitting but opaque material may be ground glass, light-transmitting cloth, or dyed plastic formed by injection molding of dyes and plastic, which is not limited in this application. The support portion 1022 may be made of a light-proof material or a light-transmitting but opaque material, to prevent the user from clearly seeing, through the support portion 1022, the component structures disposed on the first side 1011 of the lampshade body 101 while providing support for the transparent portion 1021. The support portion 1022 may be made of metal, or plastic, which is not limited in this application. The support portion 1022 is preferably made of metal. Compared with plastic, metal has higher hardness and is of a more stable molecular structure. Therefore, when the support portion 1022 is made of metal, the support portion 1022 can not only better support the transparent portion 1021, but also avoid its own degradation and aging caused by long-term exposure to the light from the sterilization lamp 400. The transparent portion 1021 is made of a colorless and transparent material to facilitate transmission of the light from the sterilization lamp 400. The colorless and transparent material may be quartz glass, transparent plastic, or transparent resin, which is not limited in this application. The light of the sterilization lamp 400 is mostly ultraviolet light. In consideration of this, in a possible implementation of this application, the transparent portion 1021 is made of a cyclic block copolymer (CBC). Compared with other materials, the transparent portion 1021 made of the cyclic block copolymer has a higher ultraviolet transmissivity. In addition, because compared with other materials, the cyclic block copolymer is of a more stable molecular structure, the transparent portion made of the cyclic block copolymer can effectively reduce a degradation speed of the transparent portion 1021 under the light from the sterilization lamp 400, prolong a service life of the lampshade 100, and ensure a sterilization effect of the sterilization lamp 400.

The lampshade body 101, the transparent portion 1021, and the support portion 1022 may be made of the same material. Specifically, in another possible implementation of this application, the lampshade body 101, the transparent portion 1021, and the support portion 1022 are all made of colorless and transparent materials. The colorless and transparent material may be quartz glass, transparent plastic, transparent resin, or acrylic, which is not limited in this application. In a possible implementation of this application, the lampshade body 101, the transparent portion 1021, and the support portion 1022 are all made of cyclic block copolymers (CBC) with more stable molecular structures, thereby avoiding premature degradation and aging of the lampshade body 101, the transparent portion 1021, and the support portion 1022 under the light from the sterilization lamp 400. Surfaces of the lampshade body 101, the transparent portion 1021, and the support portion 1022 are further coated with a light-transmitting coating, to block the line of sight of the user and prevent the user from seeing structures on the other side of the lampshade 100 through the lampshade body 101 and the support portion 1022. The light-transmitting coating may be made of an ultraviolet(UV) resistant paint or a matte transparent paint, which is not limited in this application. In a possible implementation of this application, the lampshade body 101 and the support portion 1022 are coated with the ultraviolet (UV) resistant paint, thereby further reducing adverse effects of the light from the sterilization lamp 400 on the lampshade body 101 and the support portion 1022, and improving the service life of the lampshade 100.

It may be understood that when the lampshade body 101, the transparent portion 1021, and the support portion 1022 are all made of the same material, the lampshade body 101, the transparent portion 1021, and the support portion 1022 may be integrally formed or separately disposed, which is not limited in this application. In a possible implementation of this application, the lampshade body 101, the transparent portion 1021, and the support portion 1022 are integrally formed, thereby reducing manufacturing costs of the lampshade 100.

The recessed structure 102 may have various shapes. Refer to FIG. 3. FIG. 3 is a first embodiment of the recessed structure 102. For ease of description, in this embodiment, the transparent portion 1021, the support portion 1022, and the lampshade body 101 are all considered as plate structures with flat surfaces. The lampshade body 101 is constructed as a first plate body. The transparent portion 1021 is constructed as a third plate body. The support portion 1022 is constructed as a second plate body. Based on this, in this embodiment, the transparent portion 1021, the support portion 1022, and an extension line of the lampshade body 101 jointly enclose a triangular structure. A second included angle A is formed between the lampshade body 101 constructed as the first plate body and the transparent portion 1021 constructed as the third plate body. It may be understood that if the second included angle A is too small, the light emitted by the sterilization lamp 400 is not easy to be transmitted outward from the transparent portion 1021. If the second included angle A is too large, the transparent portion 1021 may be seen by the user. Therefore, in a possible implementation of this application, the second included angle A satisfies 60°≤A≤150°. When A=90°, it can be ensured that the transparent portion 1021 is located within the visual blind region of the user, and the light from the sterilization lamp 400 can pass through. In this way, when the user looks at the lampshade body 101 from a point S, the user can only see the support portion 1022 connected to the lampshade body 101, but cannot see the transparent portion 1021 located behind the lampshade body 101, thereby preventing the user from clearly seeing, through the transparent portion 1021, component structures disposed on the first side 1011 of the lampshade body 101.

In a possible implementation of this application, a first included angle B is formed between the first plate body formed by the lampshade body 101 and the second plate body formed by the support portion 1022. When 90°≤B≤160°, the support portion 1022 can not only support the transparent portion 1021 well, but also ensure that the light from the sterilization lamp 400 is normally transmitted outward from the transparent portion 1021.

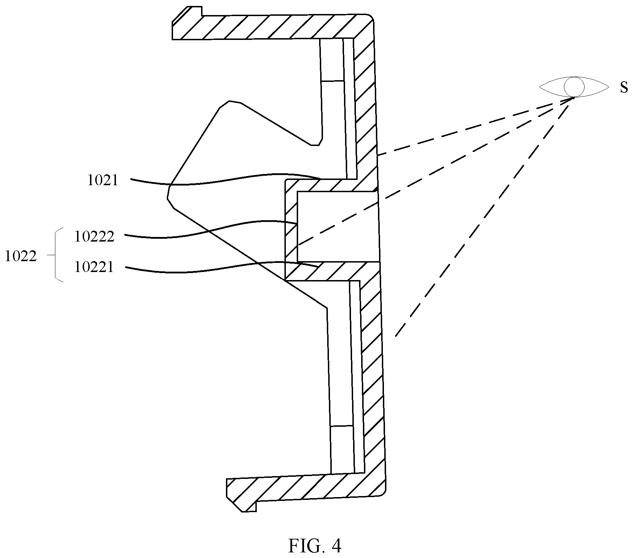

Refer to FIG. 4. FIG. 4 is a second embodiment of the recessed structure 102. In this embodiment, the support portion 1022 includes a first support plate 10221 and a second support plate 10222. One end of the first support plate 10221 is connected to the lampshade body 101, and the other end is connected to one end of the second support plate 10222. The other end of the second support plate 10222 is connected to the transparent portion 1021. The transparent portion 1021, the support portion 1022, and the extension line of the lampshade body 101 jointly enclose a rectangular structure. Similarly, in this embodiment, the second included angle A formed by the transparent portion 1021 and the lampshade body 101 satisfies 60°≤A≤150°. In this way, when the user looks at the lampshade body 101 from the point S, the user can only see the support portion 1022 connected to the lampshade body 101 and cannot see the transparent portion 1021 located behind the lampshade body 101.

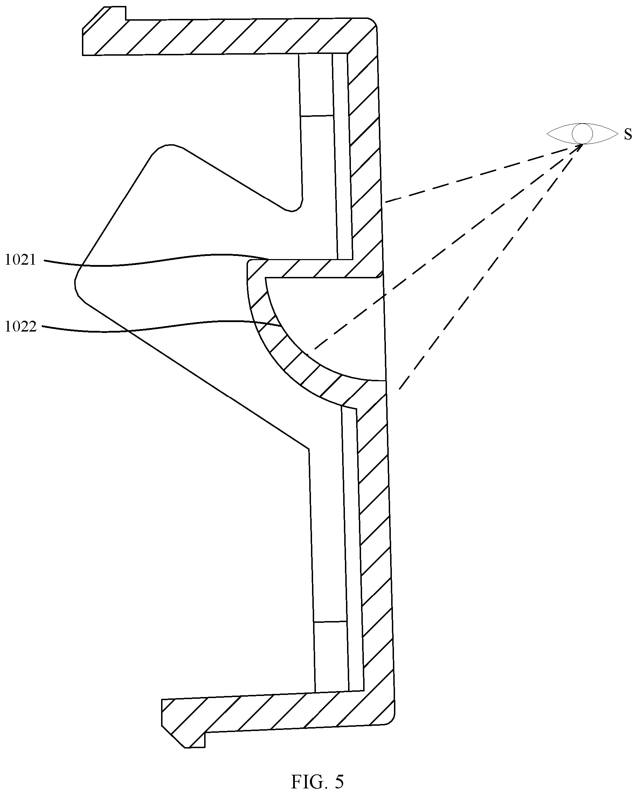

Refer to FIG. 5. FIG. 5 is a third embodiment of the recessed structure 102. In this embodiment, the support portion 1022 is of an arc-shaped structure. The transparent portion 1021, the support portion 1022, and the extension line of the lampshade body 101 jointly enclose an arc-shaped structure. In this embodiment, the second included angle A formed by the transparent portion 1021 and the lampshade body 101 satisfies 60°≤A≤150°. In this way, when looking at the lampshade body 101 from the point S, the user also cannot see the transparent portion 1021 behind the lampshade body 101.

The lampshade 100 provided by this application may have various application scenarios. For example, refer to FIG. 6. In a possible implementation of this application, the lampshade 100 may be used in the lamp assembly 1000. The lamp assembly 1000 includes: a base 200, a sterilization lamp 400, and a lampshade 100. The lampshade 100 is fastened to the base 200. The first side 1011 of the lampshade body 101 of the lampshade 100 is disposed corresponding to the base 200. The lampshade 100 and the base 200 jointly enclose a cavity 300. The sterilization lamp 400 is disposed in the cavity 300. Light from the sterilization lamp 400 is transmitted outward from the transparent portion 1021 of the lampshade 100.

In this embodiment, the lampshade 100 is disposed on the base 200 of the lamp assembly 1000. The lampshade 100 and the base 200 jointly enclose the cavity 300. The sterilization lamp 400 is disposed in the cavity 300. The light from the sterilization lamp 400 is transmitted outward from the transparent portion 1021 of the lampshade 100. However, due to the presence of the recessed structure 102, the user cannot see, through the transparent portion 1021 from a normal perspective, other internal constructions of the lamp assembly 1000, thereby improving an aesthetic appearance of the lamp assembly 1000.

The sterilization lamp 400 is configured to emit sterilization light outward to kill external bacteria. The sterilization lamp 400 may be a straight-tube hot-cathode low-pressure mercury ultraviolet lamp, an ozone ultraviolet lamp, or a UV ultraviolet lamp, which is not limited in this application.

It may be understood that the lamp assembly 1000 may further include an illumination lamp 500. The illumination lamp 500 is configured to emit illumination light for daily illumination. The illumination lamp may be an incandescent lamp or an LED lamp, which is not limited in this application.

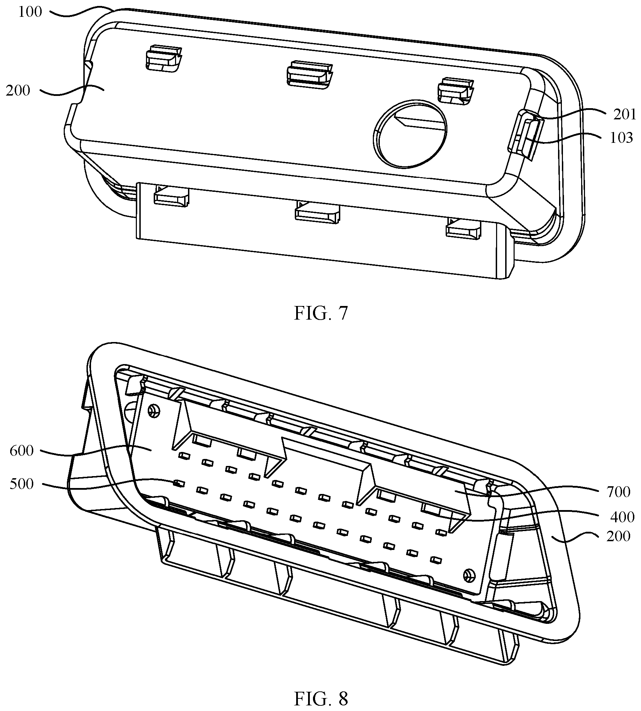

The base 200 is configured to form a space for mounting the sterilization lamp 400. The base 200 is provided with a mounting opening. The sterilization lamp 400 is mounted in the base 200 through the mounting opening. The base 200 may be made of metal, or plastic, which is not limited in this application. The lampshade 100 covers the base 200. The lampshade 100 may be connected to the base 200 in various manners. For example, the lampshade 100 may be connected to the base 200 in a threaded manner, or may be connected to the base 200 in a magnetically attractable manner, which is not limited in this application. Refer to FIG. 7. In a possible implementation of this application, the lampshade 100 further includes a plurality of snap-fit joints 103. The snap-fit joints 103 are disposed on the first side 1011 of the lampshade body 101 and are fastened to the lampshade body 101. The base 200 includes slots 201. The snap-fit joints 103 of the lampshade 100 are snap-fitted into the slots 201 of the base 200, to fasten the lampshade 100 onto the base 200. Compared with other connection manners, the use of the snap-fit joints 103 for the lampshade 100 may reduce manufacturing costs of the lampshade 100, and improve assembly efficiency between the lampshade 100 and the base 200, thereby saving costs.

Refer to FIG. 6 and FIG. 8. The lamp assembly 1000 further includes a circuit board 600. The circuit board 600 is connected to the base 200 via threads, magnetic attraction, or other fastening manners. The circuit board 600 is connected to a power supply. The illumination lamp 500 and the sterilization lamp 400 are electrically connected to the circuit board 600. The arrangement of the circuit board 600 can organize wiring of the illumination lamp 500 and the sterilization lamp 400 in the cavity 300, reducing a quantity of wiring harnesses used to electrically connect the illumination lamp 500 and the sterilization lamp 400, and reducing assembly costs. In addition, the circuit board 600 may provide support for the arrangement of the sterilization lamp 400. By changing an arrangement direction of the circuit board 600 in the cavity 300, an irradiation direction of the sterilization lamp 400 may be changed.

It may be understood that if an included angle between the sterilization lamp 400 and the transparent portion 1021 is too large, ultraviolet light emitted by the sterilization lamp 400 cannot reach the transparent portion 1021. If the included angle is too small, light transmitted out of the transparent portion 1021 may be directly refracted on the support portion 1022, and cannot be transmitted out of the recessed structure 102. Therefore, in a possible implementation manner of this application, an included angle is formed between the lampshade body 101 and the circuit board 600. To facilitate the description of beneficial effects of this embodiment, an example in which outer contours of both the lampshade body 101 and the circuit board 600 are configured as plate-type structures with flat surfaces is used for description. In this structure, the lampshade body 101 is constructed as a first plate body. A third included angle C is formed between the circuit board 600 and the lampshade body 101. When 10°≤C≤60°, the transparent portion 1021 exhibits the best refraction effect and the largest refraction range for ultraviolet light emitted by the sterilization lamp 400.

To prevent the light of the sterilization lamp 400 from reaching the support portion 1022 and causing degradation of the support portion 1022, in a possible implementation of this application, an included angle is formed between the lampshade body 101 and the support portion 1022. To facilitate the description of beneficial effects of this embodiment, an example in which outer contours of both the lampshade body 101 and the support portion 1022 are configured as plate-type structures with flat surfaces is used for description. In this structure, the lampshade body 101 is constructed as a first plate body. The support portion 1022 is constructed as a second plate body. A fourth included angle D is formed between the circuit board 600 and the support portion 1022. When 60°≤D≤120°, it can be ensured that the light from the sterilization lamp 400 is transmitted outward, while also ensuring that the light emitted by the sterilization lamp 400 does not reach the support portion 1022.

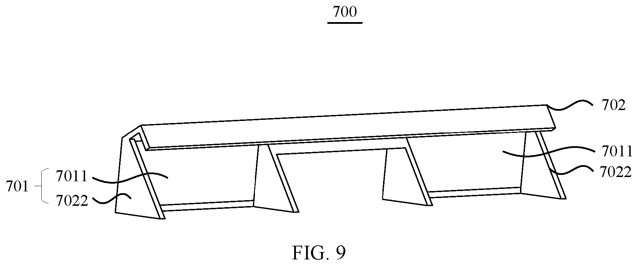

Refer to FIG. 8 and FIG. 9. The lamp assembly 1000 further includes a light baffle 700. The light baffle 700 is fastened to the circuit board 600. The light baffle 700 is disposed between the sterilization lamp 400 and the lampshade body 101, to block light from the sterilization lamp 400 to the lampshade body 101, thereby preventing the light from the sterilization lamp 400 from reaching the lampshade body 101 and reacting with the lampshade body 101, causing degradation and yellowing of the lampshade body 101.

In a possible implementation of this application, one end of the light baffle 700 is in contact with the transparent portion 1021. It should be explained that the statement that one end of the light baffle 700 is in contact with the transparent portion 1021 means that the light baffle 700 is completely fitted to the transparent portion 1021 without transmitting a force to the transparent portion 1021, which prevents the light from the sterilization lamp 400 from leaking outward from a contact point between the light baffle 700 and the transparent portion 1021 without damaging the transparent portion 1021, thereby improving a blocking effect of the light baffle 700 on the light from the sterilization lamp 400.

The light baffle 700 and the circuit board 600 may be fastened in various manners. The light baffle 700 may be directly screwed to the circuit board 600, or may be fastened to the circuit board 600 through magnetic attraction, which is not limited in this application. In a possible implementation of this application, the light baffle 700 includes a hook portion 702 and a blocking portion 701 connected to the hook portion 702. The blocking portion 701 is configured to block light from the sterilization lamp 400 to the lampshade body 101. The hook portion 702 is configured to allow the light baffle 700 to be hooked to the circuit board 600. Compared with other mounting manners, a manner of mounting the light baffle 700 on the circuit board 600 via the hook portion 702 is simpler, and higher in mounting efficiency.

To block the light, in a possible implementation of this application, the blocking portion 701 includes a first blocking portion 7011 and at least two second blocking portions 7022. The first blocking portion 7011 and the second blocking portions 7022 may be integrally formed, or may be separately disposed, which is not limited in this application. The first blocking portion 7011 and the second blocking portions 7022 jointly enclose at least one barrier space, and the sterilization lamp 400 is disposed in the barrier space, to block light from the sterilization lamp 400 to the lampshade body 101.

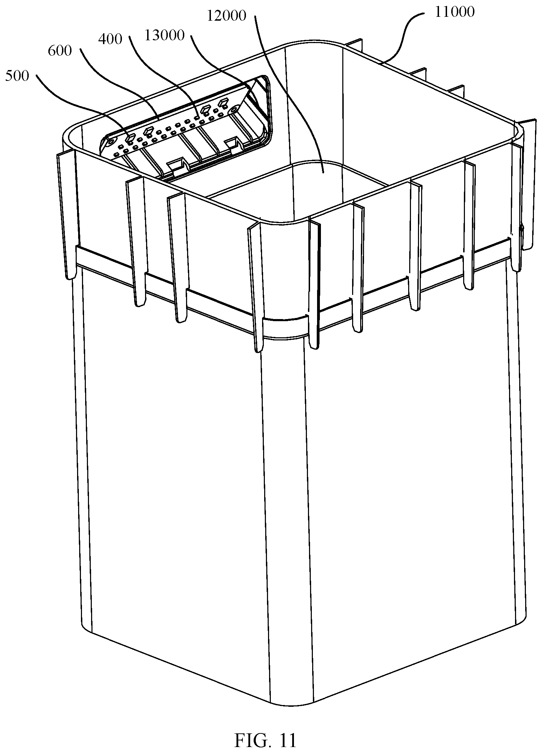

Refer to FIG. 10 and FIG. 11, the lampshade 100 provided by this application may be further used in the vehicle-mounted refrigerator 10000. In some possible implementations of this application, the lampshade 100 may be disposed on the vehicle-mounted refrigerator 10000 via the lamp assembly 1000. Specifically, the vehicle-mounted refrigerator 10000 includes a refrigerator housing 11000 and the lamp assembly 1000. A refrigerating cavity 12000 is formed in the refrigerator housing 11000. A mounting hole 13000 penetrating through the refrigerator housing 11000 is formed in an inner cavity surface of the refrigerating cavity 12000. The lampshade 100 of the lamp assembly 1000 covers the mounting hole 13000.

It may be understood that the vehicle-mounted refrigerator 10000 should further include a refrigerating system. The refrigerating system is configured to exchange heat at the refrigerating cavity 12000, to generate cold air in the refrigerating cavity 12000. Because the refrigerating system belongs to a common technical means for persons skilled in the art, it will not be described in detail in this application.

In this embodiment, the refrigerating cavity 12000 of the vehicle-mounted refrigerator 10000 is configured to store food or other items requiring refrigeration. The lamp assembly 1000 is configured to kill bacteria on the food or items stored in the refrigerating cavity 12000. The sterilization lamp 400 of the lamp assembly 1000 may emit light through the transparent portion 1021 to the refrigerating cavity 12000. Due to the recessed structure 102 on the lampshade 100, the user cannot clearly see internal constructions of the lamp assembly 1000 through the transparent portion 1021 from a normal prospective, thereby improving an overall aesthetic appearance of the vehicle-mounted refrigerator 10000.

It may be understood that in some other possible implementations of this application, the lampshade 100 may be directly disposed on the vehicle-mounted refrigerator 10000, which can prevent the internal constructions of the vehicle-mounted refrigerator 10000 from being seen by the user while allowing light in the vehicle-mounted refrigerator 10000 to pass through, improving the overall aesthetic appearance of the vehicle-mounted refrigerator 10000. It should be noted that the light in the vehicle-mounted refrigerator 10000 may be emitted by an ultraviolet lamp, an infrared lamp, or colored lamps of other colors, which is not limited in this application.

The lampshade 100 provided by this application may be further used in a vehicle. Specifically, in some embodiments of this application, this application provides a vehicle. The vehicle includes a vehicle-mounted refrigerator 10000, and the vehicle-mounted refrigerator 10000 includes the lampshade 100 or a lamp assembly 1000 with the lampshade 100.

In some embodiments of this application, the vehicle provided by this application may further include a lamp assembly 1000. The lamp assembly 1000 includes a lampshade 100. Light from a sterilization lamp 400 of the lamp assembly 1000 may be transmitted outward from the lampshade 100 to the interior of the vehicle for sterilization and disinfection. However, due to the presence of the recessed structure 102 of the lampshade 100, the user cannot clearly see the internal constructions of the lamp assembly 1000 from the outside of the lamp assembly 1000, thereby improving an overall aesthetic appearance of the vehicle.

In some embodiments of this application, the lampshade 100 provided by this application may be directly disposed in the vehicle, which can prevent internal constructions of the vehicle from being seen by the user, while allowing light in the vehicle to pass through. It should be noted that the light in the vehicle may be emitted by an ultraviolet lamp, an infrared lamp, or colored lamps of other colors, which is not limited in this application.

In the descriptions of this application, it should be noted that orientations or positional relationships indicated by the terms "center", "upper", "lower", "left", "right", "vertical", "horizontal", "inner", "outer", "circumferential", and the like are orientations or positional relationships in the drawings, and are only for the purpose of facilitating and simplifying the descriptions of the embodiments of this application instead of indicating or implying that apparatuses or elements indicated must have particular orientations, and be constructed and operated in the particular orientations, so that these terms are not construed as limiting this application.

The foregoing disclosure is merely a preferred embodiment of this application, and should not be construed as limiting the scope of the claims of this application. Persons of ordinary skill in the art may understand that implementing all or part of the processes of the foregoing embodiment and making equivalent changes according to the claims of this application still fall within the scope of this application.

## Claims

1. A lampshade (100), wherein the lampshade (100) comprises a lampshade body (101), the lampshade body (101) has a first side (1011) and a second side (1012) that are opposite to each other, the lampshade (100) comprises a recessed structure (102) that is recessed toward the first side (1011), the recessed structure (102) comprises a transparent portion (1021), and the transparent portion (1021) is capable of transmitting light from a sterilization lamp (400) disposed on the first side (1011) outward from the second side (1012).

2. The lampshade according to claim 1, wherein the transparent portion (1021) is made of a colorless and transparent material.

3. The lampshade according to claim 1, wherein the lampshade body (101) is made of a light-transmitting but opaque material, and the transparent portion is made of quartz glass or a cyclic block copolymer.

4. The lampshade according to claim 1, wherein both the lampshade body (101) and the transparent portion (1021) are made of colorless and transparent materials, and the lampshade body (101) is coated with a light-transmitting coating.

5. The lampshade according to claim 4, wherein the lampshade body (101) is made of acrylic or a cyclic block copolymer, and the light-transmitting coating is an anti-ultraviolet coating.

6. The lampshade according to any one of claims 1 to 5, wherein the lampshade body (101) and the recessed structure (102) are integrally formed.

7. The lampshade according to any one of claims 1 to 6, wherein the recessed structure (102) further comprises a support portion (1022), and the support portion (1022) is separately connected to the lampshade body (101) and the transparent portion (1021) to support the transparent portion (1021).

8. The lampshade according to claim 7, wherein one end of the transparent portion (1021) is connected to the lampshade body (101), and the other end is connected to the support portion (1022.)

9. The lampshade according to claim 7 or 8, wherein the lampshade body (101) is constructed as a first plate body, the support portion (1022) is constructed as a second plate body, a first included angle B is formed between the first plate body and the second plate body, and 90°≤B≤160°.

10. The lampshade according to any one of claims 1 to 9, wherein the lampshade body (101) is constructed as the first plate body, the transparent portion (1021) is constructed as a third plate body, a second included angle A is formed between the first plate body and the third plate body, and 60°≤A≤150°.

11. The lampshade according to any one of claims 1 to 10, wherein the lampshade (100) further comprises a plurality of snap-fit joints (103), and the snap-fit joints (103) are disposed on the first side (1011) of the lampshade body (101) and fastened to the lampshade body (101).

12. A lamp assembly (1000), comprising:
a base (200);
a sterilization lamp (400), connected to the base (200); and
the lampshade (100) according to any one of claims 1 to 11, the lampshade (100) is fastened to the base (200), and the first side (1011) of the lampshade body (101) of the lampshade (100) is disposed corresponding to the base (200), the lampshade (100) and the base (200) jointly enclose a cavity (300), the sterilization lamp (400) is disposed in the cavity (300), and light from the sterilization lamp (400) is transmitted outward from the transparent portion (1021) of the lampshade (100).

13. The lamp assembly according to claim 12, wherein the lamp assembly (1000) further comprises a circuit board (600), the circuit board (600) is mounted in the cavity (300), and the sterilization lamp (400) is disposed on the circuit board (600).

14. The lamp assembly according to claim 13, wherein the lampshade body (101) is constructed as a first plate body, a third included angle C is formed between the circuit board (600) and the lampshade body (101), and 10°≤C≤60°.

15. The lamp assembly according to claim 13 or 14, wherein the recessed structure (102) further comprises the support portion (1022), the support portion (1022) is separately connected to the lampshade body (101) and the transparent portion (1021) to support the transparent portion (1021), the support portion (1022) is constructed as a second plate body, a fourth included angle D is formed between the circuit board (600) and the support portion (1022), and 60°≤D≤120°.

16. The lamp assembly according to any one of claims 13 to 15, wherein the lamp assembly (1000) further comprises a light baffle (700), the light baffle (700) is fastened to the circuit board (600), and the light baffle (700) is disposed between the sterilization lamp (400) and the lampshade body (101).

17. The lamp assembly according to claim 16, wherein one end of the light baffle (700) is in contact with the transparent portion (1021).

18. The lamp assembly according to claim 16 or 17, wherein the light baffle (700) comprises a hook portion (702) and a blocking portion (701) connected to the hook portion (702), and the hook portion (702) is hooked to the circuit board (600).

19. A vehicle-mounted refrigerator (10000), wherein the vehicle-mounted refrigerator (10000) comprises:
a refrigerator housing (11000), a refrigerating cavity (12000) is formed in the refrigerator housing (11000), and the refrigerator housing (11000) is provided with a mounting hole (13000); and
the lamp assembly (1000) according to any one of claims 12 to 18, the lamp assembly (1000) is mounted in the mounting hole (13000).

20. A vehicle, wherein the vehicle comprises the lampshade (100) according to any one of claims 1 to 11, comprises the lamp assembly (1000) according to any one of claims 12 to 18, or comprises the vehicle-mounted refrigerator (10000) according to claim 19.
